# EUROPEAN PATENT SPECIFICATION

(11) **EP 4 058 111 B1**
(45) Date of publication and mention of the grant of the patent: **02.04.2025**
(21) Application number: 20799701.6
(22) Date of filing: 30.10.2020
(51) Int. Cl.: A61M 5/32

(54) **A MEDICAMENT DELIVERY DEVICE**
MEDIKAMENTENABGABEVORRICHTUNG
DISPOSITIF D'ADMINISTRATION DE MÉDICAMENTS

(30) Priority: 14.11.2019 EP 19209132
(43) Date of publication of application: 21.09.2022
(73) Proprietor: SHL Medical AG, 6302 Zug (CH)
(72) Inventor: KARLSSON, Anders, 131 28 Nacka Strand (SE)
(86) International application number: PCT/EP2020/080526
(87) International publication number: WO 2021/094108

(56) References cited:
- WO-A1-2015/113172
- WO-A1-2017/144200
- WO-A1-2018/172223

## Description

### TECHNICAL AREA

The present application relates to a medicament delivery device specifically a medicament delivery device with an accidental activation mechanism.

### BACKGROUND OF INVENTION

Medicament delivery devices such as auto-injectors, pen-injectors, inhalers, on-body devices are generally known for the self administration of a medicament by patients without formal medical training occurs. As just one example, those patients suffering from diabetes may require repeated injections of insulin. Other patients may require regular injections of other types of medicaments, such as a growth hormone or biological medicaments. Therefore, to facilitate the patients, there is a demand to design medicament delivery devices with multiple automatic functions, predetermined dosage and assembled a medicament container.

To simplify the medicament delivery operation sequence or prevent an unintentional activation of the medicament delivery operation sequence, most of medicament delivery devices in the market are designed that the medicament delivery operation sequence is activated when the medicament delivery device is aimed to the medicament delivery site; more particularly, a medicament delivery member is aimed to the medicament delivery site.

Also, most of medicament delivery devices in the market are provided with cover members configured to bi-directionally moved in relation to medicament delivery members to cover medicament delivery members, e.g. needle, catheter or nozzle; before and after an operation of medicament delivery devices, the bi-directional movement of cover members usually will be restricted after the operation of medicament delivery devices, e.g. after injection; such that the injury or the infection by accessing delivery members before or after the operation of medicament delivery devices will also be prevented.

Since the movement of the cover member is usually associated with the medicament delivery member, arranging the medicament delivery operation sequence being activated in response to the movement of the cover member of the medicament delivery device, can be a guarantee that the medicament delivery operation sequence is activated when the medicament delivery member is aimed to the medicament delivery site.

Such function and/ or arrangement is commonly used in medicament delivery devices in the market, and it can be achieved by, for example, a mechanism described in any one of WO 2018/172223, WO 02/47746, WO 2011/005177 and WO 2015/113172.

The document WO 02/47746 discloses a medicament delivery device provided with an activator tube and a needle cover. A medicament delivery operation sequence of the medicament delivery device is activated by the distal axial movement of the needle cover and followed by the proximal axial movement of the activator tube.

The document WO 2011/005177 discloses a medicament delivery device provided with a second activator member and a needle shield sleeve. A medicament delivery operation sequence of the medicament delivery device is activated by both needle shield sleeve moving into a distal position and the second activator member moving into a proximal position.

The document WO 2015/113172 discloses a medicament delivery device provided with a housing, a needle guard and a protective cap. The needle guard is configured to activate a medicament delivery operation sequence of the medicament delivery device by moving into the housing.

However, there is a risk of accidental activation of the medicament delivery operation sequence of the medicament delivery devices which is caused by dropping the medicament delivery devices or shaking during transporting the medicament delivery devices to an end user. Therefore, there is a demand of arranging an accidental activation prevention design on such medicament delivery devices.

The document WO 2015/113172 discloses an accidental activation prevention mechanism is arranged between the housing, the needle guard and the protective cap. The needle guard comprises a locking means arranged with a resilient portion, a contact surface and a cam. The protective cap is configured to deflect the locking means when it is attached to the housing, such that the contact surface is opposite to an abutment on the housing, the rearward movement of the needle guard is therefore prevented.

However, the accidental activation prevention mechanism provided by WO 2015/113172, using the interaction between the contact surface on a resilient tongue and the abutment on the housing to restrict the axial movement of the needle guard, especially during the dropping or shaking the medicament delivery device, the resilient tongue is therefore subjected to a compressive axial stress. Such that a buckling may occur on the resilient tongue. The buckling may cause a deformation or breakage of the resilient tongue and consequentially block the movement of the needle guard during activating the medicament delivery device.

### SUMMARY

The aim of the present invention is to solve or at least mitigate the problem of the prior art.

In the present disclosure, when the term "distal direction" is used, this refers to the direction pointing away from the dose delivery site during use of the medicament delivery device. When the term "distal part/end" is used, this refers to the part/end of the delivery device, or the parts/ends of the members thereof, which under use of the medicament delivery device is/are located furthest away from the dose delivery site. Correspondingly, when the term "proximal direction" is used, this refers to the direction pointing towards the dose delivery site during use of the medicament delivery device.

When the term "proximal part/end" is used, this refers to the part/end of the delivery device, or the parts/ends of the members thereof, which under use of the medicament delivery device is/are located closest to the dose delivery site.

Further, the terms "longitudinal", "longitudinally", "axially" and "axial" refer to a direction extending from the proximal end to the distal end and along the device or components thereof, typically in the direction of the longest extension of the device and/or component.

Similarly, the terms "transverse", "transversal" and "transversally" refer to a direction generally perpendicular to the longitudinal direction.

An object of this invention is to provide a simple and reliable way of accidental activation prevention mechanism for a medicament delivery device.

According to an aspect of the invention, the object is achieved by a robust and reliable accidental activation prevention mechanism according to claim 1. There is hence provided a medicament delivery device comprising: a tubular housing comprising a distal end and a proximal end; a medicament container arranged with a medicament delivery member and placed in the housing, a cap removably arranged on the proximal end of the housing and configured to cover the delivery member; a drive mechanism configured to act on the medicament container to expel the medicament through the medicament delivery member upon the drive mechanism is triggered; an activation member arranged coaxially movable in relation to the housing, associated with the drive mechanism and comprising a proximal tubular portion which extends from the proximal end of the housing and wherein for triggering the drive mechanism the activation member is axially moved towards the distal end of the housing after removing the cap from the housing; the proximal tubular portion of the activation member comprises a resilient element arranged with a friction member; and the cap comprises an interaction member configured to radial force the resilient element and thereby causes the friction member to laterally engage with the housing when the cap is arranged on the proximal end of the housing; whereby an accidental axial movement of the activation member is prevented.

The accidental activation prevention mechanism is hence obtained by means of the lateral friction-fitting instead of relying on an axially stressed resilient tongue configuration as in WO 2015/113172. Instead of applying an abutting force on an edge of a resilient tongue that may cause the buckling, the lateral friction is evenly borne by the whole or major portion on the lateral surface of the resilient element, such that the buckling can be prevented. Furthermore, the accidental activation prevention mechanism is more often to be performed by a mechanical form-fitting arrangement, namely the activation member is fitted to the housing by the shaped or structural abutments on the activation member and a portion of the housing, such as latch or the abutment and the counter abutment like in WO 2015/113172; that is usually required a space for accommodating that form-fitting arrangement. The lateral friction-fitting arrangement may reduce the required space, since the retaining friction is controlled by whether there is a pressing force applied on the resilient element, such that the medicament delivery device can be more compact.

According to one embodiment, the friction member is laterally arranged on resilient element and is configured to interact with the inner surface of the proximal portion of the housing.

According to the invention, the proximal tubular portion comprises a lateral cut-out; wherein the cut-out defines the resilient element with a fixed end and a free end which is radially movable in relation to the proximal tubular portion; and the friction member is arranged on the outer surface of the free end of the resilient element.

According to one embodiment, the proximal tubular portion comprises a lateral arranged pair of slots; wherein the pair of slots defines the resilient element; and wherein the friction member is arranged on the outer surface of the resilient element.

According to one embodiment, the friction member is radial outwardly protruding from resilient element and the proximal tubular portion.

According to the invention, the outer surface of the proximal tubular portion where the resilient element and the friction member is located, is at least partially surrounded by the inner surface of the proximal portion of the housing.

According to one embodiment, the interaction member of the cap is coaxially arranged within the proximal tubular portion.

According to one embodiment, the activation member is configured to trigger the drive mechanism when the activation member axially moves towards the distal end of the housing.

According to one embodiment, the medicament delivery device is further comprises a trigger button arranged on the distal end of the housing and movable between a distal initial position to a proximal trigger position; wherein the drive mechanism is triggered only when the trigger button is in its proximal trigger position and the activation member is axially moved towards the distal end of the housing.

According to one embodiment, the trigger button is prevented from moving into its proximal trigger position until the activation member is axially moved towards the distal end of the housing.

According to one embodiment, the proximal tubular portion is configured to completely surround the medicament delivery member when the activation member extends from the proximal end of the housing and the cap is removed from the proximal end of the housing.

According to one embodiment, the interaction member is coaxially arranged with the medicament delivery member when the cap is arranged on the proximal end of the housing.

According to one embodiment, the medicament container can be axially fixed to the housing of the medicament delivery device; or axially movable within the housing of the medicament delivery device.

According to one embodiment, the medicament container can be a syringe arranged with a needle or a delivery nozzle; or a cartridge that can be attached with a needle or a delivery nozzle by an end user.

According to one embodiment, the medicament delivery device can be an injection device, an inhalation device, an on-body device or a medical sprayer.

Other aspects, features, and advantages will be apparent from the summary above, as well as from the description that follows, including the figures and the claims.

Generally, all terms used in the claims are to be interpreted according to their ordinary meaning in the technical field, unless explicitly defined otherwise herein. All references to "a/an/the element, apparatus, component, means, etc. are to be interpreted openly as referring to at least one instance of the element, apparatus, component, means, etc.", unless explicitly stated otherwise.

### BRIEF DESCRIPTION OF THE DRAWINGS

The specific embodiments of the inventive concept will now be described, by way of example, with reference to the accompanying drawings, in which:
Fig. 1 displays a medicament delivery device.
Fig. 2A-2B display an activation member with a proximal tubular portion which is axially movable in relation to the housing of the medicament delivery device of Fig.1.
Fig. 3A-3B display the proximal tubular portion of the activation member of the medicament delivery device of Fig.1.
Fig. 4 displays a cap of the medicament delivery device of Fig.1.
Fig. 5-6 display cross-section views of a mechanical arrangement of the accidental activation prevention of the medicament delivery device of Fig.1.
Fig. 7 displays a cross-section view of the proximal tubular portion of the activation member when the cap is moved from the of the medicament delivery device of Fig.1.
Fig. 8A-8B display cross-section views of the medicament delivery device of Fig.1 arranged with and without the cap.

### DETAILED DESCRIPTION

The inventive concept will now be described more fully hereinafter with reference to the accompanying drawings, in which exemplifying embodiments are shown. The inventive concept may, however, be embodied in many different forms and should not be construed as limited to the embodiments set forth herein; rather, these embodiments are provided by way of example so that this disclosure will be thorough and complete, and will fully convey the scope of the inventive concept to those skilled in the art. Like numbers refer to like elements throughout the description.

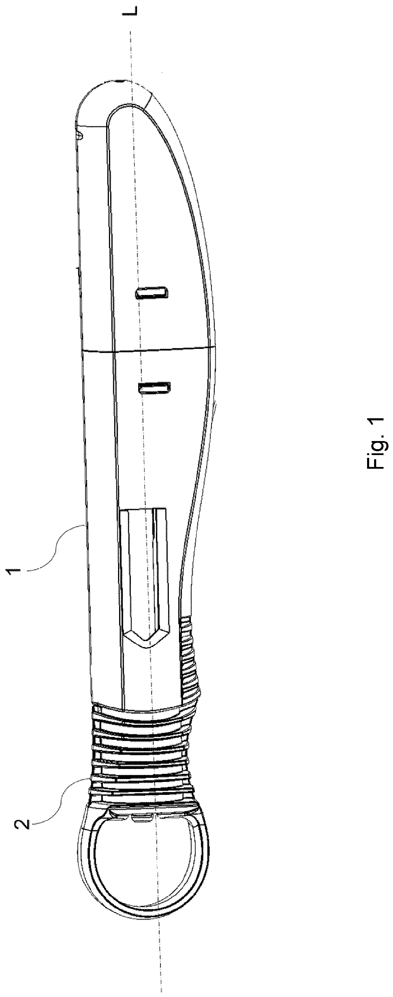

Fig.1 illustrates a medicament delivery device with a tubular housing 1 which extends longitudinally along an axis L from a proximal end to a distal end; and a cap 2 arranged on the proximal end of the tubular housing 1. The tubular housing 1 is configured to accommodate a medicament container 5 containing a medicament and having a medicament delivery member 51 which is arranged on the proximal end of the medicament container 5 and arranged to deliver the medicament to an end user of the medicament delivery device.

The medicament delivery device also comprises a drive mechanism configured to act on the medicament container 5 to expel the medicament through the medicament delivery member 51 upon the drive mechanism is triggered. The drive mechanism comprises an energy source, such as a spring, a gas canister or a motor; a plunger rod which is biased by the energy source in the proximal direction and is configured to act on the medicament container 5 to expel the medicament through the medicament delivery member 51. The plunger rod may be hold against the energy source by a holding member before the drive mechanism is triggered; or the energy source may be prevented to output a force before the drive mechanism is triggered, such that the medicament can only be delivered upon the drive mechanism is triggered.

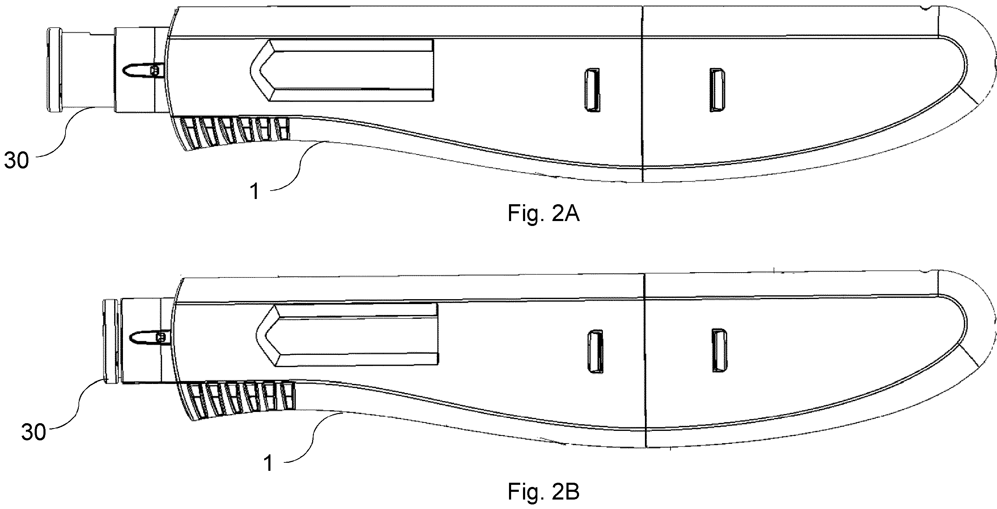

The medicament delivery device further comprises an activation member 3 arranged coaxially movable in relation to the housing 1 and associated with the drive mechanism. The activation member 3 comprises a proximal tubular portion 30 that extends from the proximal end of the housing 1 as shown in Fig. 2A. For triggering the drive mechanism, the activation member 3 is axially moved towards the distal end of the housing 1 by distally moving the proximal tubular portion 30, such as pressing the proximal end of the proximal tubular portion 30 onto the medicament delivery site, at least partially into the housing 1 as shown in Fig. 2B. The distal portion of the activation member 3 (not show) is configured to interact with the drive mechanism upon the distal movement of the activation member 3. Such as the distal portion of the activation member may move the holding member out from the engagement with the plunger rod; or release the holding member from the engagement with the plunger rod; or release the power source to output the force toward the plunger rod. The drive mechanism is thereby triggered.

The activation member 3 may be further associated with a trigger button (not show). The trigger button may be arranged on the distal end of the housing or on the lateral outer surface of the housing 1. The trigger button is movable between an initial position to an axially proximal trigger position or a transverse trigger position. The drive mechanism is arranged to be triggered only when the trigger button is in its axial proximal trigger position or traverse trigger position and the activation member is axially moved towards the distal end of the housing 1. The trigger button may be prevented from moving into its axial proximal trigger position or traverse trigger position until the activation member 3 is axially moved towards the distal end of the housing 1.

Since this aspect does not form part of the present disclosure, and since many different variations of such drive mechanism associated with the distal movement of the activation member and/ or the movement of the trigger button can be realised, such as those drive mechanism disclosed in the cited prior art documents, this feature will not be discussed in any further detail herein.

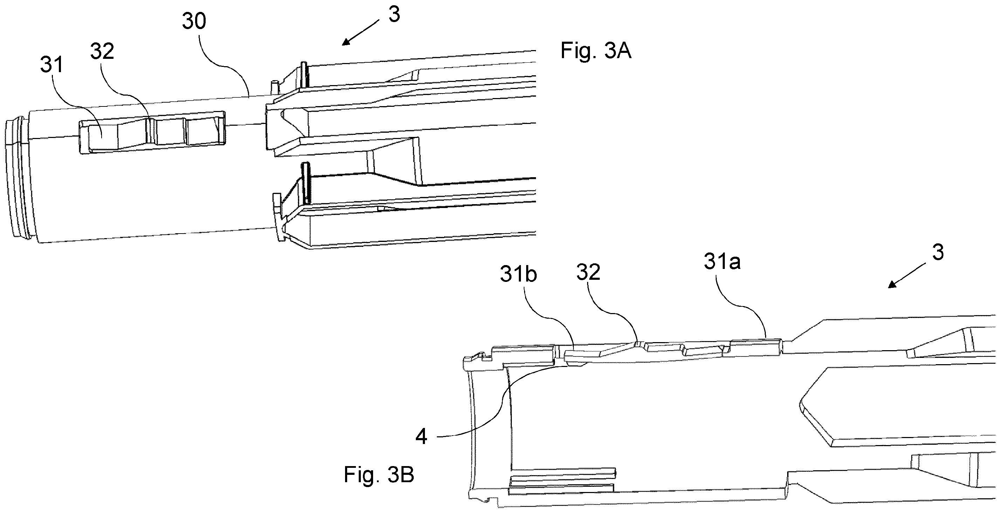

Fig. 3A-3B illustrate the proximal tubular portion 30 of the activation member 3 of the medicament delivery device. The proximal tubular portion 30 comprises a resilient element 31, which is formed by a cut-out on the proximal tubular portion 30. A friction member 32 protrudes radially outwards from the lateral outer surface of the resilient element 31. The friction member 32 is configured to laterally interact with the inner surface 11 of the housing 1, such that the proximal tubular portion 30 is friction-fitted with housing 1 through the friction member 32. The friction member 32 may comprise a rough surface or a rubber surface to engage with the inner surface 11 of the housing 1 for increasing the friction between the friction member 32 and the housing 1. The friction member 32 may also comprise a chamfer surface for distributing the normal force evenly around interface between the friction member 32 and the inner surface 11 of the housing 1. The resilient element 31 comprises a fixed end 31a which is fixed to the proximal tubular portion 30; and a free end 31b which is radially movable in relation to the proximal tubular portion 30. The friction member 32 is arranged close to the free end 31a as shown in Fig. 3B. The inner surface of the free end 31b is arranged with an interaction protrusion 4 configured to interact with the cap 2.

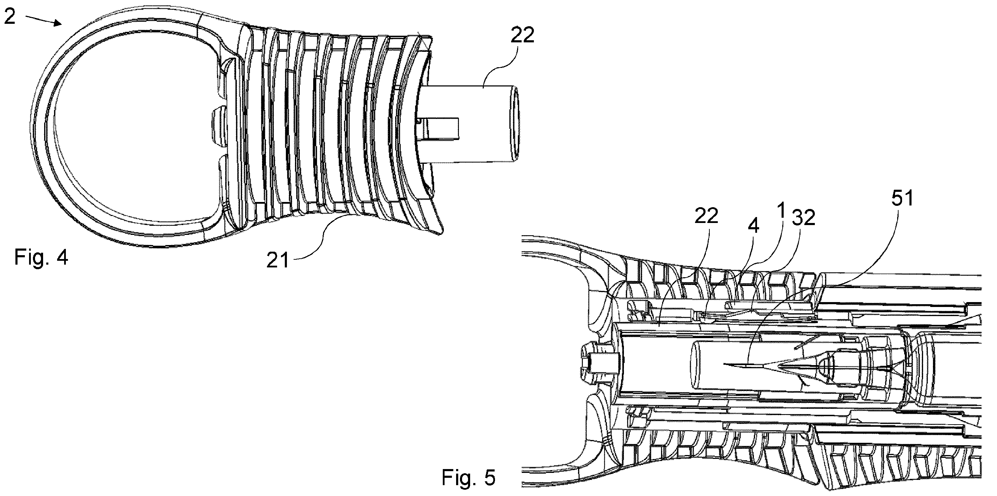

Fig. 4 illustrates a cap 2 comprising an outer cap body 21 which is accessible by the end user; and an interaction member 22 arranged within the outer cap body 21. The interaction member 22 is configured to contact with the interaction protrusion 4 when the cap 2 is attached to the housing 1, the resilient element 31 is thereby forced radially outwards by the interaction member 22 such that the friction member 32 tightly engages with the inner surface 11 of the housing.

The interaction member 22 is configured to provide a pressing force or normal force on the resilient portion, so that the friction for retaining the proximal tubular portion 30 can be generated when the cap 2 is attached to the housing 1.

It should be noted that alternatively, the interaction protrusion can be rearranged onto the interaction member of the cap, and configured to contact the flat inner surface of the resilient element of the proximal tubular portion. It is also feasible that the interaction protrusion can be removed, since the interaction member can be arranged with a slightly greater diameter than the inner diameter of the proximal tubular portion, such that the interaction member is compressed and assembled into the proximal tubular portion, and therefore the interaction member will provide the pressing force to the resilient element. To increase the friction generated by the friction member 32 and the inner surface 11 of the housing 1, it is preferred that the interaction member 22 creates less deformation on the resilient element 31; such that the pressing force from the interaction member 22 can be mainly transited into the pressing force or the normal force on the resilient element 31.

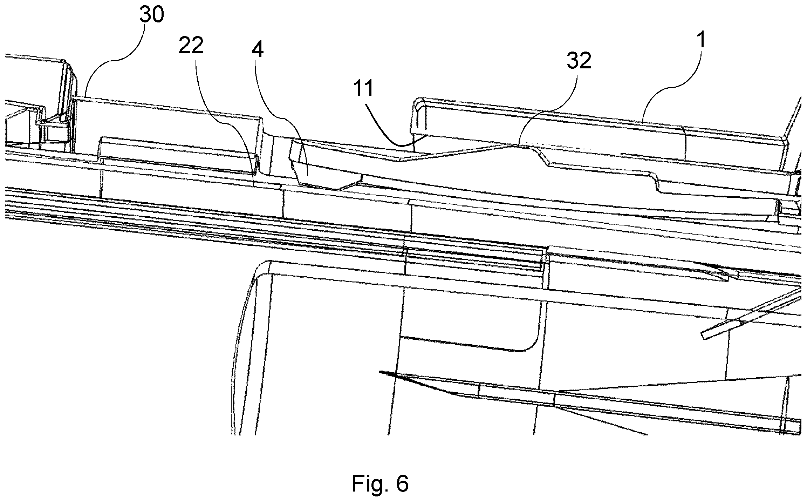

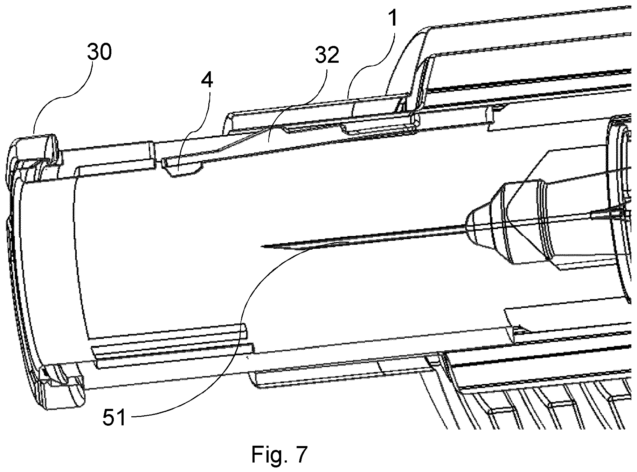

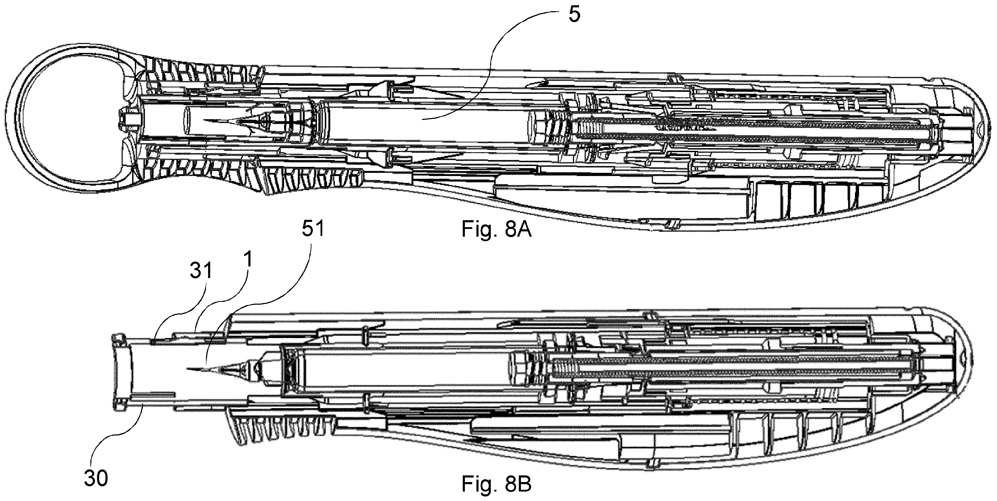

Fig. 5-6 and Fig. 8A illustrates the mechanical arrangement of the accidental activation prevention of the invention. When the cap 2 is attached to the housing 1, the interaction member 22 provides a constant bending force to the resilient element 31 through the contact with the interaction protrusion 4. The bending force provides the pressing force or the normal force to the friction member 32, such that the friction member 32 is friction-fitted to the inner surface 11 of the housing 1. An accidental axial movement of the activation member 3 is thereby prevented. When the cap 2 is detached from the housing 1, as shown in Fig. 7 and Fig. 8B, since there is no any support on the inner surface of the resilient element 31, the pressing force to the friction member 32 is also dismissed. The friction between the friction member 32 and the inner surface 11 of the housing 1 is therefore significantly reduced and is no longer able to fix the proximal tubular portion 30 and the activation member 3 to the housing 1. Such that the end user is able to trigger the drive mechanism by distally moving the activation member 3 to start the medicament delivery operation sequence.

In an example not being part of the invention, instead of the cut-out, the resilient element 31 can be defined by a lateral arranged pair of slots on the proximal tubular portion 30. Such as the free end 31b as described above can be rearranged as a second fixed end, and the friction member 32 can be arranged between the two fixed ends.

In an example not being part of the invention, the friction member 32 can also be alternatively arranged on the inner surface of the resilient element 31. Such arrangement is suitable for a housing with two layers proximal portion. In this embodiment, the proximal tubular portion is arranged between the outer layer housing and the inner layer housing; the interaction member of the cap is configured to contact the interaction protrusion or directly to the outer surface of the resilient element, such that the friction member is engaged with the outer surface of the inner layer of the housing and friction-fitted with housing.

In an example not being part of the invention, the friction member 32, instead of protruding from either the inner or the outer surface of the resilient element 31, can be alternatively flat arranged on the inner or the outer surface of the resilient element 31. Similarly, the interaction protrusion 4 can also be removed, such that the interaction member 22 can directly contact on the inner or outer surface of the resilient element 31.

The proximal tubular portion 30 also acts as the delivery member cover, such that the proximal tubular portion 30 is configured to completely surround the medicament delivery member 51 when the activation member 3 extends from the proximal end of the housing 1 and the cap 2 is removed from the proximal end of the housing 1, as shown in Fig. 7 and Fig. 8B.

The interaction member 22 of the cap 2 can also serve as or be used with a delivery member sheath remover. Such that the interaction member 22 is coaxially arranged with the medicament delivery member 51 when the cap 2 is arranged on the proximal end of the housing 1, as shown in Fig. 5-6 and Fig. 8A. The inner surface of the interaction member may be arranged with a gripper configured to grip on the medicament delivery member sheath; or a delivery member sheath remover comprising the grapper may be arranged within the interaction member. Therefore, the delivery member sheath can be removed with the removal of the cap 2 from the housing 1. If the cap 2 serves as or be used with a delivery member sheath remover, the friction between the delivery member and the delivery member sheath may cause the cap 2 can only be removed while a significant pulling force is applied to the cap 2. Therefore, the interface between the interaction member 22 and the interaction protrusion 4 can be smooth and provided with less friction. On the other hand, if the cap 2 is free from the function of delivery member sheath remover; or to more strongly keep the proximal tubular portion 3 immovable when the cap 2 is attached to the housing 1, the interface between the interaction member 22 and the interaction protrusion 4 can be also arranged with high friction, such as rough interface or rubber interface; therefore the proximal tubular portion 3 and so as the activation member can be friction-fitted with both the housing 1 and the cap 2.

The inventive concept has been described above with reference to a few examples. However, as is readily appreciated by a person skilled in the art, other embodiments than the ones disclosed above are equally possible within the scope of the inventive concept, as defined by the appended claims.

## Claims

1. A medicament delivery device comprising:
- a tubular housing (1) comprising a distal end and a proximal end;
- a medicament container (5) arranged with a medicament delivery member (51) and placed in the housing (1);
- a cap (2) removably arranged on the proximal end of the housing (1) and configured to cover the delivery member (51);
- a drive mechanism configured to act on the medicament container (5) to expel the medicament through the medicament delivery member (51) upon the drive mechanism being triggered;
- an activation member (3) arranged coaxially movable in relation to the housing, associated with the drive mechanism and comprising a proximal tubular portion (30) which extends from the proximal end of the housing (1) and wherein for triggering the drive mechanism the activation member (3) is axially moved towards the distal end of the housing (1) after removing the cap (2) from the housing (1);
the proximal tubular portion (30) of the activation member (3) comprising a resilient element (31) arranged with a friction member (32); and the cap (2) comprising an interaction member (22) configured to radially force the resilient element (31) and thereby cause the friction member (32) to laterally engage with the housing (1) when the cap (2) is arranged on the proximal end of the housing (1); whereby an accidental axial movement of the activation member (3) is prevented;
wherein the proximal tubular portion (30) comprises a lateral cut-out; wherein the cut-out defines the resilient element (31) with a fixed end (31a) and a free end (31b) which is radially movable in relation to the proximal tubular portion (30); and wherein the friction member (32) is arranged on the outer surface of the free end (31b) of the resilient element (31);
wherein the outer surface of the proximal tubular portion (30) where the resilient element (31) and the friction member (32) are located, is at least partially surrounded by the inner surface (11) of the proximal portion of the housing (1);
wherein when the cap (2) is attached to the housing (1), the interaction member (22) is configured to provide a pressing force or normal force on the resilient element (31), so that the friction for retaining the proximal tubular portion (30) is generated.

2. The medicament delivery device according to claim 1, wherein the friction member (32) is laterally arranged on the resilient element (31) and is configured to interact with the inner surface (11) of the proximal portion of the housing (1).

3. The medicament delivery device according to claim 1 or 2, wherein the proximal tubular portion (30) comprises a laterally arranged pair of slots; wherein the pair of slots defines the resilient element (31); and wherein the friction member (32) is arranged on the outer surface of the resilient element (31).

4. The medicament delivery device according to any one of the preceding claims 1-3, wherein the friction member (32) is radial outwardly protruding from the resilient element (31) and the proximal tubular portion (30).

5. The medicament delivery device according to anyone of the preceding claims 1-4, wherein the interaction member (22) of the cap (2) is coaxially arranged within the proximal tubular portion (30).

6. The medicament delivery device according to claim 1, wherein the activation member (3) is configured to trigger the drive mechanism when the activation member (3) axially moves towards the distal end of the housing.

7. The medicament delivery device according to claim 1, wherein the medicament delivery device further comprises a trigger button arranged on the housing (1) and movable between an initial position to a trigger position; wherein the drive mechanism is triggered only when the trigger button is in its trigger position and the activation member (3) is axially moved towards the distal end of the housing (1).

8. The medicament delivery device according to claim 7, wherein the trigger button is prevented from moving into its trigger position until the activation member (3) is axially moved towards the distal end of the housing (1).

9. The medicament delivery device according to claim 1, wherein the proximal tubular portion (30) is configured to completely surround the medicament delivery member (51) when the activation member (3) extends from the proximal end of the housing (1) and the cap (2) is removed from the proximal end of the housing (1).

10. The medicament delivery member according to claim 1, wherein the interaction member (22) is coaxially arranged with the medicament delivery member (51) when the cap (2) is arranged on the proximal end of the housing (1).

## Patentansprüche

1. Medikamentenabgabevorrichtung, umfassend:
- ein röhrenförmiges Gehäuse (1), umfassend ein distales und ein proximales Ende;
- einen Medikamentenbehälter (5), der mit einem Medikamentenabgabeelement (51) angeordnet und in dem Gehäuse (1) platziert ist;
- eine Kappe (2), die an dem proximalen Ende des Gehäuses (1) entfernbar angeordnet und konfiguriert ist, um das Abgabeelement (51) abzudecken;
- einen Antriebsmechanismus, der konfiguriert ist, um auf den Medikamentenbehälter (5) einzuwirken, um das Medikament durch das Medikamentenabgabeelement (51) auszustoßen, wenn der Antriebsmechanismus ausgelöst wird;
- ein Aktivierungselement (3), das in Bezug auf das Gehäuse koaxial bewegbar angeordnet ist, das dem Antriebsmechanismus zugeordnet ist und umfassend einen proximalen röhrenförmigen Abschnitt (30), der sich von dem proximalen Ende des Gehäuses (1) erstreckt, und wobei zum Auslösen des Antriebsmechanismus das Aktivierungselement (3) in Richtung des distalen Endes des Gehäuses (1) nach dem Entfernen der Kappe (2) von dem Gehäuse (1) axial bewegt wird;
der proximale röhrenförmige Abschnitt (30) des Aktivierungselements (3) umfassend ein elastisches Element (31), das mit einem Reibungselement (32) angeordnet ist; und die Kappe (2) umfassend ein Interaktionselement (22), das konfiguriert ist, um das elastische Element (31) radial zu drängen und dadurch zu bewirken, dass das Reibungselement (32) seitlich mit dem Gehäuse (1) in Eingriff kommt, wenn die Kappe (2) an dem proximalen Ende des Gehäuses (1) angeordnet ist; wodurch eine unbeabsichtigte axiale Bewegung des Aktivierungselements (3) verhindert wird;
wobei der proximale röhrenförmige Abschnitt (30) einen seitlichen Ausschnitt umfasst;
wobei der Ausschnitt das elastische Element (31) mit einem festen Ende (31a) und einem freien Ende (31b) definiert, das in Bezug auf den proximalen röhrenförmigen Abschnitt (30) radial bewegbar ist; und wobei das Reibungselement (32) auf der Außenoberfläche des freien Endes (31b) des elastischen Elements (31) angeordnet ist;
wobei die Außenoberfläche des proximalen röhrenförmigen Abschnitts (30), an dem sich das elastische Element (31) und das Reibungselement (32) befinden, mindestens teilweise durch die Innenoberfläche (11) des proximalen Abschnitts des Gehäuses (1) umgeben ist;
Wobei, wenn die Kappe (2) an dem Gehäuse (1) angebracht ist, das Interaktionselement (22) konfiguriert ist, um eine Druckkraft oder Normalkraft auf das elastische Element (31) auszuüben, sodass die Reibung zum Festhalten des proximalen röhrenförmigen Abschnitts (30) erzeugt wird.

2. Medikamentenabgabevorrichtung nach Anspruch 1, wobei das Reibungselement (32) an dem elastischen Element (31) seitlich angeordnet und konfiguriert ist, um mit der Innenoberfläche (11) des proximalen Abschnitts des Gehäuses (1) zu interagieren.

3. Medikamentenabgabevorrichtung nach Anspruch 1 oder 2, wobei der proximale röhrenförmige Abschnitt (30) ein seitlich angeordnetes Paar von Schlitzen umfasst; wobei das Paar von Schlitzen das elastische Element (31) definiert; und wobei das Reibungselement (32) auf der Außenoberfläche des elastischen Elements (31) angeordnet ist.

4. Medikamentenabgabevorrichtung nach einem der vorstehenden Ansprüche 1 bis 3, wobei das Reibungselement (32) von dem elastischen Element (31) und dem proximalen röhrenförmigen Abschnitt (30) radial nach außen vorsteht.

5. Medikamentenabgabevorrichtung nach einem der vorstehenden Ansprüche 1 bis 4, wobei das Interaktionselement (22) der Kappe (2) innerhalb des proximalen röhrenförmigen Abschnitts (30) koaxial angeordnet ist.

6. Medikamentenabgabevorrichtung nach Anspruch 1, wobei das Aktivierungselement (3) konfiguriert ist, um den Antriebsmechanismus auszulösen, wenn sich das Aktivierungselement (3) in Richtung des distalen Endes des Gehäuses axial bewegt.

7. Medikamentenabgabevorrichtung nach Anspruch 1, wobei die Medikamentenabgabevorrichtung ferner einen Auslöseknopf umfasst, der an dem Gehäuse (1) angeordnet und zwischen einer Ausgangsposition und einer Auslöseposition bewegbar ist; wobei der Antriebsmechanismus nur ausgelöst wird, wenn sich der Auslöseknopf in seiner Auslöseposition befindet und das Aktivierungselement (3) in Richtung des distalen Endes des Gehäuses (1) axial bewegt wird.

8. Medikamentenabgabevorrichtung nach Anspruch 7, wobei der Auslöseknopf daran gehindert wird, sich in seine Auslöseposition zu bewegen, bis das Aktivierungselement (3) in Richtung des distalen Endes des Gehäuses (1) axial bewegt wird.

9. Medikamentenabgabevorrichtung nach Anspruch 1, wobei der proximale röhrenförmige Abschnitt (30) konfiguriert ist, um das Medikamentenabgabeelement (51) vollständig zu umgeben, wenn sich das Aktivierungselement (3) aus dem proximalen Ende des Gehäuses (1) erstreckt und die Kappe (2) von dem proximalen Ende des Gehäuses (1) entfernt ist.

10. Medikamentenabgabeelement nach Anspruch 1, wobei das Interaktionselement (22) mit dem Medikamentenabgabeelement (51) koaxial angeordnet ist, wenn die Kappe (2) an dem proximalen Ende des Gehäuses (1) angeordnet ist.

## Revendications

1. Dispositif d'administration de médicament comprenant :
- un boîtier tubulaire (1) comprenant une extrémité distale et une extrémité proximale ;
- un récipient de médicament (5) agencé avec un élément d'administration de médicament (51) et placé dans le boîtier (1) ;
- un capuchon (2) agencé de manière amovible sur l'extrémité proximale du boîtier (1) et conçu pour recouvrir l'élément d'administration (51) ;
- un mécanisme d'entraînement conçu pour agir sur le récipient de médicament (5) pour expulser le médicament par l'intermédiaire de l'élément d'administration de médicament (51) lorsque le mécanisme d'entraînement est déclenché ;
- un élément d'activation (3) agencé coaxialement se déplaçant par rapport au boîtier, associé au mécanisme d'entraînement et comprenant une partie tubulaire proximale (30) qui s'étend de l'extrémité proximale du boîtier (1) et dans lequel pour déclencher le mécanisme d'entraînement l'élément d'activation (3) est déplacé axialement vers l'extrémité distale du boîtier (1) après le retrait du capuchon (2) du boîtier (1) ;
la partie tubulaire proximale (30) de l'élément d'activation (3) comprenant un élément élastique (31) agencé avec un élément de frottement (32) ; et le capuchon (2) comprenant un élément d'interaction (22) conçu pour forcer radialement l'élément élastique (31) et de ce fait amener l'élément de frottement (32) à venir en prise latéralement avec le boîtier (1) lorsque le capuchon (2) est agencé sur l'extrémité proximale du boîtier (1) ; moyennant quoi un mouvement axial accidentel de l'élément d'activation (3) est empêché ;
dans lequel la partie tubulaire proximale (30) comprend une découpe latérale ;
dans lequel la découpe définit l'élément élastique (31) avec une extrémité fixe (31a) et une extrémité libre (31b) qui peut être déplacée radialement par rapport à la partie tubulaire proximale (30) ; et dans lequel l'élément de frottement (32) est agencé sur la surface externe de l'extrémité libre (31b) de l'élément élastique (31) ;
dans lequel la surface externe de la partie tubulaire proximale (30) où sont positionnés l'élément élastique (31) et l'élément de frottement (32), est au moins partiellement entourée par la surface interne (11) de la partie proximale du boîtier (1) ;
dans lequel lorsque le capuchon (2) est fixé au boîtier (1), l'élément d'interaction (22) est conçu pour fournir une force de pression ou une force normale sur l'élément élastique (31), de sorte que le frottement pour retenir la partie tubulaire proximale (30) est généré.

2. Dispositif d'administration de médicament selon la revendication 1, dans lequel l'élément de frottement (32) est agencé latéralement sur l'élément élastique (31) et est conçu pour interagir avec la surface interne (11) de la partie proximale du boîtier (1).

3. Dispositif d'administration de médicament selon la revendication 1 ou 2, dans lequel la partie tubulaire proximale (30) comprend une paire de fentes agencées latéralement ; dans lequel la paire de fentes définit l'élément élastique (31) ; et dans lequel l'élément de frottement (32) est agencé sur la surface externe de l'élément élastique (31).

4. Dispositif d'administration de médicament selon l'une quelconque des revendications 1 à 3 précédentes, dans lequel l'élément de frottement (32) fait saillie radialement vers l'extérieur de l'élément élastique (31) et de la partie tubulaire proximale (30).

5. Dispositif d'administration de médicament selon l'une quelconque des revendications 1 à 4 précédentes, dans lequel l'élément d'interaction (22) du capuchon (2) est agencé coaxialement au sein de la partie tubulaire proximale (30).

6. Dispositif d'administration de médicament selon la revendication 1, dans lequel l'élément d'activation (3) est conçu pour déclencher le mécanisme d'entraînement lorsque l'élément d'activation (3) se déplace axialement vers l'extrémité distale du boîtier.

7. Dispositif d'administration de médicament selon la revendication 1, dans lequel le dispositif d'administration de médicament comprend en outre un bouton de déclenchement agencé sur le boîtier (1) et pouvant se déplacer entre une position initiale jusqu'à une position de déclenchement ; dans lequel le mécanisme d'entraînement est déclenché uniquement lorsque le bouton de déclenchement est dans sa position de déclenchement et que l'élément d'activation (3) est déplacé axialement vers l'extrémité distale du boîtier (1).

8. Dispositif d'administration de médicament selon la revendication 7, dans lequel le bouton de déclenchement est empêché de se déplacer dans sa position de déclenchement jusqu'à ce que l'élément d'activation (3) soit déplacé axialement vers l'extrémité distale du boîtier (1).

9. Dispositif d'administration de médicament selon la revendication 1, dans lequel la partie tubulaire proximale (30) est conçue pour entourer complètement l'élément d'administration de médicament (51) lorsque l'élément d'activation (3) s'étend de l'extrémité proximale du boîtier (1) et que le capuchon (2) est retiré de l'extrémité proximale du boîtier (1).

10. Élément d'administration de médicament selon la revendication 1, dans lequel l'élément d'interaction (22) est agencé coaxialement avec l'élément d'administration de médicament (51) lorsque le capuchon (2) est agencé sur l'extrémité proximale du boîtier (1).
